# EUROPEAN PATENT APPLICATION

(11) **EP 2 757 537 A1**
(43) Date of publication of application: **23.07.2014**
(21) Application number: 13152012.4
(22) Date of filing: 21.01.2013
(51) Int. Cl.: G07F 11/68, A61J 7/04, G06F 19/00, G07F 17/00

(54) **Medication dispenser**

(71) Applicant: Evondos Oy, 24240 Salo (FI)
(72) Inventor: Apell, Mika, 20700 Turku (FI); Niinistö, Jyrki, 24800 Halikko (FI)
(74) Representative: Turun Patenttitoimisto Oy

(57) **Abstract**

The present invention provides a medication dispenser (100), comprising a first container (110) for holding medication packages (120) that contain medications to be taken at predetermined taking times, and conveying means (140, 150) for conveying medication packages (120) from the first container (110) to an outlet (130, 131) of the medication dispenser (100) at the predetermined taking times. The medication dispenser (100) further comprises a camera (170) for monitoring the operation of the medication dispenser (100), the camera (170) being arranged to image a plurality of objects at different locations inside the medication dispenser (100).

## Description

### TECHNICAL FIELD OF THE INVENTION

The present invention relates to a medication dispenser, a system and a method for monitoring the operation of a medication dispenser according to the preambles of the appended independent claims.

### BACKGROUND OF THE INVENTION

Various devices are used for assisting patients in complying with their medical regimens. The most elementary ones are handled fully manually, and the most sophisticated devices are so-called medication dispensers, which dispense proper dosages of medications at prescribed times. The medications are prepackaged by licensed pharmacies into medication packages, which are provided with labels that may contain information about the patient, the content of the package, and the taking time of the dosage. Typically, the medication packages are connected together to form a strip, from which medication packages are dispensed one by one.

In a known medication dispenser, medication packages, which have been inserted into a container of the medication dispenser, are arranged to be delivered to a patient one package at a time according to the information provided in the labels of the packages. The medication dispenser notifies the patient when it is time to take the medications. If the patient acknowledges the notification, the medication package is conveyed out of the medication dispenser. Otherwise, the medication package is kept in the medication dispenser.

A problem associated with the known medication dispenser relates to defects in its medical safety and reliability. Patient access is typically needed for the handling of medications to be dispensed. The patient has access also to other medications than the one he should take at a certain time. Thus, he may take more than one medication and overdose. On the other hand, medication handling in automated dispensers is not always reliable and the patient needs support to correct unrecognised problems in the medication handling. This takes time from the caregivers since they need to visit the patient's home and spend time finding out the problem and resolving it.

### OBJECTIVES OF THE INVENTION

It is the main objective of the present invention to reduce or even eliminate the prior art problems presented above.

It is an objective of the present invention to provide a method for dispensing medications and a medication dispenser, which increase the medical safety and medication dispensing reliability compared to the prior art. In more detail, it is an objective of the invention to provide a method for dispensing medications and a medication dispenser enabling to efficiently control the taking of medications, while being able to efficiently and in a simple manner monitor different targets and different medication package layouts inside the medication dispenser. It is a further objective of the invention to provide a method for dispensing medications and a medication dispenser enabling to reduce the probability of taking medications at a wrong time, or by someone else than the patient.

In order to realise the above-mentioned objectives, the dispenser, system and method according to the invention are characterised by what is presented in the characterising parts of the appended independent claims. Advantageous embodiments of the invention are described in the dependent claims.

### DESCRIPTION OF THE INVENTION

A typical medication dispenser comprises
- a first container for holding medication packages that contain medications to be taken at predetermined taking times, and
- conveying means for conveying medication packages from the first container to an outlet of the medication dispenser at the predetermined taking times.

It further comprises
- a camera for monitoring the operation of the medication dispenser, the camera being arranged to image a plurality of objects at different locations inside the medication dispenser.

The present invention thus provides for a medication dispenser, wherein the handling and recognising of medications is performed inside an automated dispenser, thus preventing unnecessary or unauthorised access to the medications. The complexity, cost and reliability concerns are addressed by the camera arranged to image a plurality of objects at different locations inside the medication dispenser, allowing the monitoring of different targets with one single camera for different purposes to input data for the actuator control.

Furthermore, a camera is a reliable and flexible multipurpose sensor, with a high processing power, while the development of different image processing algorithms enable the handling of the information for different purposes. The high automation level thus gives the patients a much appreciated easiness of use.

The conveying means may be one or more conveyors (such as two, three or four conveyors) known as such. The conveyors may thus comprise for example rollers or belts. The conveying means may comprise a first conveyor arranged in connection with the outlet and being capable of conveying a medication package in a first and a second conveying direction, so that a medication package can be conveyed to the outlet and retracted inside the medication dispenser. In the first conveying direction the medication package is conveyed towards the outlet, and in the second conveying direction the medication package is conveyed away from the outlet. Preferably, the first conveying direction is opposite to the second conveying direction. The first conveyor is thus preferably a bi-directional conveyor, which conveys a medication package to the outlet and then, in a case where the patient has not taken the medication package from the outlet or acknowledged the medication notification at all, retracts the medication package inside the medication dispenser. Preferably, the medication package is held with the first conveyor during the time the medication package is kept at the outlet.

The medication dispenser is arranged to dispense medication packages to provide the patient with the proper dosages of medications at prescribed times. The medication packages are conveyed with the conveying means from the first container to the outlet from which the patient can take the medication packages. The conveying means are typically driven by means of at least one actuator, such as an electric motor.

The medication packages usable in the present dispenser can be for example a strip of packages, bags or mugs made of plastic and/or blister packages made of plastic or metal foil. Typically each package has a label with identifying information.

The advantages of the present invention are thus that a camera enables monitoring very different actuators to give feedback for driving mechanisms, recognising the medication packages and their position, medication package information and recognising particles like medications inside the dispenser. Additionally, the handling of medications is done automatically and reliably inside the device without giving unnecessary or unauthorised access to medications which could lead to medication errors or cause danger e.g. for children.

According to an embodiment, the medication dispenser comprises moving means for moving the camera between a plurality of positions inside the medication dispenser. The position of the camera can thus be in any direction within the medication dispenser, and the camera may be arranged to be moved along one, two or three axis. The camera may also be arranged to turn around an axis. By a plurality of positions it is here meant at least two positions, but also three, four, five, six, seven or more positions.

According to an embodiment, the moving means comprises a linear actuator to which the camera is attached. The linear actuator may be a belt conveyor, a screw drive, a magnetic actuator or a combination of a rail and an electric motor, for example. There may also be two or more linear actuators, such as two, three, four, five or six linear actuators. The use of more than one linear actuator allows moving the camera in more than one direction.

According to another embodiment, the medication dispenser comprises rotating means for rotating the camera to different directions. The rotating means can consist for example of at least one solenoid, DC motor, stepper motor or other electromechanical component. A solenoid can be used when there is no need to change the imaging angles, i.e. when only fixed angles are needed. The solenoid pulls or pushes the camera to different positions around the rotation axle. The motor can be connected directly to the rotation axle which is connected to the camera directly or by at least one gear. Similarly, the angle of the rotation axle can be changed by another actuator, solenoid, DC motor, stepper motor or other electromechanical component. This thus enables two-dimensional rotation of the camera.

The moving means may also comprise only a rotating actuator, or a combination of linear and rotating actuators as described above.

The focus of the camera may be adjustable or fixed. According to a preferred embodiment, the focus of the camera is fixed and this is achieved by a lens with sufficient depth of field performance for different monitoring purposes. On the other hand, an electrically adjustable camera can be used to have an optimal focus for different image distances.

According to yet another embodiment, the optical path between the camera and an object to be imaged comprises an adjustable optical element. The optical element can be selected from the group consisting of a mirror, a prism, a lens, an optical filter or a combination of one or more thereof.

Moreover, the optical element may also be movable in at least one direction and/or turnable about at least one axis. The optical element may thus also be arranged in connection with a linear actuator and/or rotating means, and there can be one or more linear actuators as well as one or more rotating means and rotation axis.

According to an embodiment, at a first position the camera is configured to monitor medication packages conveyed on the conveying means. At this position, the camera typically determines information on a label of a medication package. Such information can be, for example, the taking time of the medications contained in the medication package. Advantageously, at the first position, the camera is arranged in connection with the conveying means.

In said first position, the camera thus provides information which can be utilised in positioning the medication package, such as taking time. After the medication package has been positioned, the camera in its first position may be used to detect the taking of the medication package from the outlet by the patient. The camera at this position may also be arranged to detect and record the time of taking the medication package from the outlet.

According to an embodiment, at a second position the camera is configured to monitor the content of a second container of the medication dispenser, which second container is arranged to receive untaken medication packages. The second container may also be arranged to receive waste detached e.g. from the medication packages during handling. The purpose of the second position is thus to give feedback about the container of missed medications, i.e. the second container. The camera can for example see the bottom of the second container and thus it can detect if the second container is empty or not. The view of the camera can also be rotated by rotating the camera, or with the help of a separate mirror to see the level of medication packages in the second container. The camera can thus also detect if a medication package has dropped from the first conveyor to the second container, or if there is a possible overflow of the second container. The camera can also detect how full the second container is. According to another embodiment, the medication dispenser comprises a mirror via which the content of the second container is monitored.

According to a further embodiment, at a third position the camera is configured to monitor the position of a cutter of the medication dispenser. The cutter is typically set to separate a medication package from a strip of medication packages and/or to open the medication package. Preferably, the cutter is arranged in connection with the conveying means. The camera can also be used as home position sensor for the cutter, ensuring that the cutter is driven to safe home position, after each use, based on position feedback from the camera.

The camera may also be arranged to be self-positionable. Indeed, the camera can be able to find its home position and/or one of its other positions by recognising the form or shape of the mechanisms inside the medication dispenser, or various markings made in these mechanisms.

The operation of the medication dispenser is preferably controlled with a control unit. The control unit may comprise a processor that is programmed to carry out the functions that are needed to operate the medication dispenser. The control unit may comprise a memory for storing, for example, the patient information.

A taking time of the medications contained in a medication package can be read from a label of the medication package or from a memory of the medication dispenser or from the server.

The medication dispenser may also be provided with a second (third, fourth etc.) camera, that may be either stationary or movable. The medication dispenser may also be provided with various sensors for detecting the position of the medication packages, although they are typically not necessary as the same function can be performed by the camera.

The invention also relates to a method for monitoring the operation of a medication dispenser. A typical method for monitoring the operation of a medication dispenser that comprises a first container for holding medication packages that contain medications to be taken at predetermined taking times, and conveying means for conveying medication packages from the first container to an outlet of the medication dispenser at the predetermined taking times, is characterised in that it comprises monitoring the operation of the medication dispenser by imaging with a camera a plurality of objects at different locations inside the medication dispenser.

The method thus enables to dispense medications to a patient in a controlled way. The idea is to allow the patient to take the medications at the right time, while limiting the time that the medications are physically available to the patient.

According to an embodiment, the method comprises moving the camera between a plurality of positions inside the medication dispenser and/or rotating the camera to different directions. In the method, the camera can thus be moved (linearly or around one or more axis) in order to image the plurality of objects.

According to an embodiment, the method comprises capturing, at one or more of the plurality of positions, at least one image in at least one direction. The camera can also be configured to capture a plurality of images in one direction, which images are then combined into a single image, or one or more images in two or more directions. Taking several images for the driver logic from different positions and directions will increase the quality and the reliability of the feedback.

According to another embodiment, the method comprises monitoring, at a first position, medication packages conveyed on the conveying means.

According to yet another embodiment, the method comprises monitoring, at a second position, the content of a second container of the medication dispenser, which second container is arranged to receive untaken medication packages.

According to a yet further embodiment, the method comprises monitoring, at a third position, the position of a cutter of the medication dispenser.

The monitoring steps are thus performed by using the camera, which is movable inside the dispenser, to various positions. The use of the camera allows a flexible detection method, allowing different medication package layouts and different targets to be monitored.

The camera can also be used to detect particles detached from packages like medicines or pieces of package material. The camera then gives feedback to actuator drivers to drive particles off the conveyers, such as by dropping them to the second container below the conveyers.

The camera can furthermore be used to monitor the medication packages moving inside the device in different locations, e.g. in parallel rails. The same moving camera can thus detect the positions of medication packages in one or more conveyers and/or the packages can be as consecutive packages at the same strip or packages of one or more parallel strips.

In the method the medications are dispensed in medication packages by using the medication dispenser. The medication packages are inserted into a container of the medication dispenser by the patient or a caregiver of the patient, such as a nurse or a near relative. Each medication package contains medications to be taken at a prescribed time, i.e. at a taking time. The packages can be, for example, bags or mugs made of plastic, or blister packages made of plastic or metal foil. Typically, the medication packages are connected together to form a strip, from which medication packages are dispensed one package at a time according to the medical regimen of the patient. The medical regimen typically contains information about medications, their dosages and the taking times. The medication packages are arranged in the strip sequentially in time order.

One example of a possible operation of a medication dispenser according to this invention is as follows. A reader head containing a camera can be moved so that in addition to reading the information from the medication package, it can be stationed in an intermediate position for detecting the content of the second ("waste") container. In one operating position the camera is focused to the vicinity of the camera in order to read the information on the medication packages.

In a second operating position (between the strip rolls or blister/mug stacks) the optical path contains a mirror and the camera is focused to infinity in order to identify whether there are medication packages in the container. In a third operating position, the camera monitors the cutter position and gives positioning input to the processor to position the cutter. In a fourth position, the optical element detects package position and can give feedback for package movement and handling like cutting. Moreover, a part of the image can be used for own position detection of the optical element by detecting fixed markings in the mechanisms.

In the method, a time window for the taking of medications contained in a medication package is first set. The time window defines the time during which the medication package may be conveyed to the outlet of the medication dispenser. The time window is set to contain at least the prescribed time for the taking of the medications contained in the medication package, i.e. the time window contains at least the taking time of the medications. The taking time may be obtained, for example, from a label of the medication package, or from a medical regimen stored in a memory of the medication dispenser. The time window may also contain time intervals preceding and following the taking time. This means that the starting time of the time window can be set to be earlier than that of the taking time, and the ending time of the time window can be set to be later than that of the taking time. However, in many cases the time window is set to correspond to the taking time.

When the time window defined for the medication package starts, the patient is notified that it is time to take the medications. The patient is typically notified with an audible signal through a loudspeaker of the medication dispenser. However, in some cases the patient may also be notified visually using a light source or a display of the medication dispenser. It is also possible to use a separate device, which can be, for example, a bracelet that contains a vibrator to notify the patient. In a case where the patient acknowledges the notification before the time window expires, the medication package is conveyed to the outlet from which the patient may take the medication package. The notification is acknowledged via a user interface of the medication dispenser. The user interface may comprise one or more buttons, and a display, such as a touch screen. The patient may acknowledge the notification, for example, by pressing a button or a certain icon that is shown on the touch screen. If the patient identification is required to be able to take the medication package from the medication dispenser, notification can be acknowledged with a patient specific RFID or NFC tag, magnetic key or electronic ID card. The medication dispenser may comprise an RFID, NFC or magnetic reader and/or an electronic card reader slot to support identification with a personal tag.

According to an embodiment, the method comprises, if the medication package is in the medication dispenser after the time window has ended, conveying the medication package to a second container, the container of missed medications. In a case where the patient has failed to acknowledge the notification, the medication package is kept within the medication dispenser during the time window, and after the time window has ended the medication package is conveyed to said second container

According to an embodiment, the step of setting the time window for the taking of the medications contained in the medication package comprises reading a taking time of the medications from a label of the medication package, and selecting the time window to consist of the taking time, a first predetermined time interval preceding the taking time, and a second predetermined time interval following the taking time. The taking times of the medications are contained in the medical regimen of the patient. The label of the medication package contains package-related information, such as the taking time of the medications. The information may be, for example, in a form of text or a one- or two-dimensional bar code. The taking time of the medications can be a certain time instant, such as 8:00 or 10:15, or a certain time interval, such as 8:00-10:00 or 10:15-11:00. The taking time of the medications can also be expressed vaguely, such as "in the morning", or "in the evening". In this case, the expressions "in the morning" and "in the evening" are defined as certain time intervals, such as 7:00-10:00 and 19:00-22:00, respectively. The first and the second predetermined time intervals define supplementary time for the taking of the medications. The length of the first and the second predetermined time interval can vary between the medication packages and can be dependent on the taking times of the previous medication packages. The length of the first and the second predetermined time interval can be, for example, less than 10 minutes, 10-30 minutes, 30-60 minutes, or 1-2 hours. According to an embodiment, the length of the first and/or the second predetermined time interval is zero.

According to an embodiment, the method comprises detecting a time of taking the medication package from the outlet. The detected time can be used in defining a time window for the next medication package. The time when the patient takes the medication package from the outlet of the medication dispenser can be detected with a detector that is arranged in connection with the outlet, or it can be detected using the camera.

According to an embodiment, the method comprises, in a situation where the dispenser cannot solve a problem,
- taking an image of the unsolved problem,
- sending the image to a server,
- receiving a solution from the server, and
- self-checking to approve the solution.

According to an embodiment, the method thus comprises sending an image to the server in case that the internal processor of the dispenser cannot process the image or detect one or more targets in one or more positions in one or more directions. The dispenser first tries to solve the problem independently but after a predefined number of retrials, such as one, two, three or more, it sends the image to the server. Thereby more processing power may be provided by the server. If also the server cannot solve the problem, a service person is notified to solve the problem. He can see the same images taken inside the dispenser and control the mechanisms by command via the server and debug the problem remotely. Thus, he typically can solve the problem remotely and time-consuming visit to patient home can be avoided. Finally, he releases the dispenser to its own control and the dispenser does a self-check to approve the solution. In a case where the patient has not taken the medications during the time window, a message can be sent over a communications network to the server. The server is preferably arranged to forward the message to the caregiver of the patient, such as a nurse or a near relative. The message typically contains the patient's name, a description of the detected problem to be solved, and the time of the observation.

The present invention also provides for an automated medication administration system comprising a medication dispenser as described and a medication administration server. The medication administration server may for example contain the information on the taking of the medication and may assist the dispenser in case of problems. The connection between the dispenser and the server may be for example wireless. The administration system is typically used for persons with medical conditions requiring long term treatment.

The system may also comprise means for communication between the dispenser and the server. The means for communication is able to send and receive information to and from both the dispenser and the server.

The exemplary embodiments of the invention presented in this text are not interpreted to pose limitations to the applicability of the appended claims. The verb "to comprise" is used in this text as an open limitation that does not exclude the existence of also unrecited features. The features recited in the dependent claims are mutually freely combinable unless otherwise explicitly stated, among the features relating to each of the dispenser, system and method, but also to each other.

### BRIEF DESCRIPTION OF THE DRAWING

The features which are considered as characteristic of the invention are set forth in particular in the appended claims. The invention itself, however, both as to its construction and its method of operation, together with additional objects and advantages thereof, will be best understood from the following description of specific embodiments when read in connection with the accompanying drawings.
- Fig. 1: illustrates an embodiment of the present invention in a first position.
- Fig. 2: illustrates an embodiment of the present invention in a second position.
- Fig. 3: illustrates an embodiment of the present invention in a third position.
- Fig. 4: illustrates another embodiment of the present invention.

### DETAILED DESCRIPTION OF THE DRAWING

The same reference signs are used of the same or like components in different embodiments.

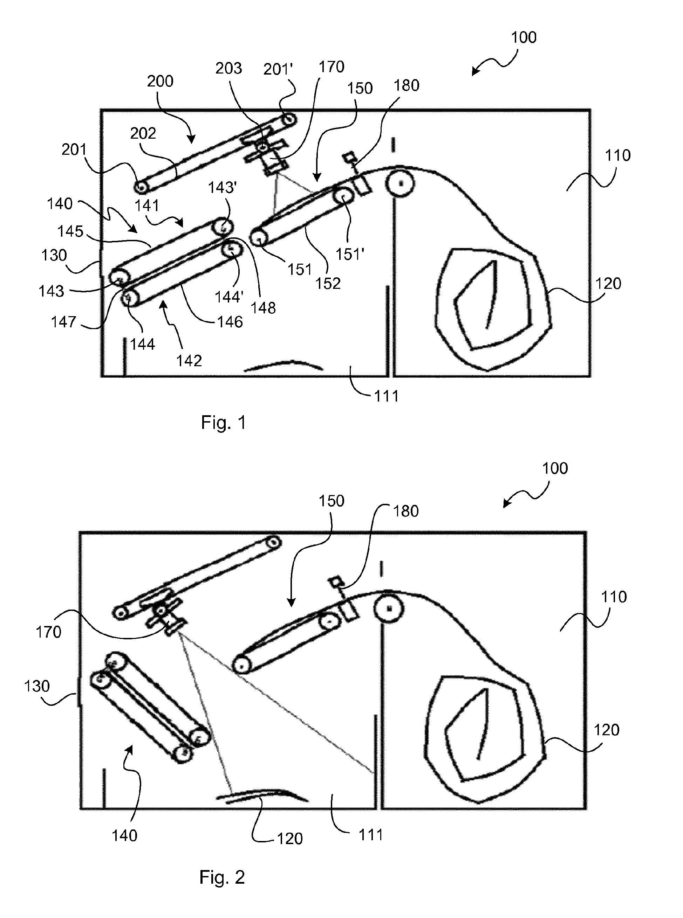

Figure 1 illustrates a sectional view of an embodiment of the present invention in a first position. The medication dispenser 100 comprises a first container 110 for holding medication packages 120 to be dispensed. The medication packages 120 form a strip from which medication packages 120 are delivered one by one to a patient. The medication packages 120 are conveyed from the first container 110 to an outlet 130 of the medication dispenser 100 with a first and a second conveyor 140, 150. The first and the second conveyor 140, 150 are provided with integrated actuators (not shown) for operating the conveyors 140, 150.

The second conveyor 150 conveys medication packages 120 from the first container 110 to the first conveyor 140. The second conveyor 150 is a belt conveyor that comprises two rollers 151, 151' surrounded by a belt 152. At the second conveyor 150, the taking time of the medications is read from a label of the medication package 120 using a camera 170. The taking time is used in setting a time window that defines the time during which the medication package 120 may be conveyed to the outlet 130. After reading the label, the medication package 120 is separated from the strip by a cutter 180.

The camera 170 is arranged on a belt conveyor 200 comprising two rollers 201, 201' surrounded by a belt 202. The camera 170 also comprises a base 203 arranged to move along the belt conveyor 200. In Figure 1, the camera is at its first position, i.e. it monitors the medication packages 120 conveyed. The camera is not tilted or rotated.

The first conveyor 140 comprises two opposing belt conveyors 141, 142 between which a medication package 120 is conveyed. Both of the belt conveyors 141, 142 comprise two rollers 143, 143', 144, 144' surrounded by a belt 145, 146. The belt conveyors 141, 142 are tied to each other with springs 147, 148. The belt conveyors 141, 142 can be rotated in both directions, whereby a medication package 120 between them may be conveyed in two opposite conveying directions.

The first conveyor 140 is turnable about an axis between a first and a second position. Referring to Fig. 1, there is shown a situation where the first conveyor 140 is at the first position. At this position, the medication package 120 can be conveyed from the second conveyor 150 to the first conveyor 140.

Figure 2 illustrates a sectional view of an embodiment of the present invention in a second position, and there is shown a situation where the first conveyor 140 is turned to the second position. Depending on the conveying direction of the first conveyor 140, the medication package 120 can be conveyed either to the outlet 130 or to a second container 111. In the situation of Figure 2, the patient has not acknowledged the notification concerning the start of the time window, and the medication package 120 has been conveyed with the first conveyor 140 to the second container 111.

In Figure 2, the camera 170 is in its second position, i.e. configured to monitor the content of the untaken medication packages. As can be seen, the camera is slightly tilted in order to have a better imaging view to the second container 111. The camera may also be rotated to allow a better imaging view.

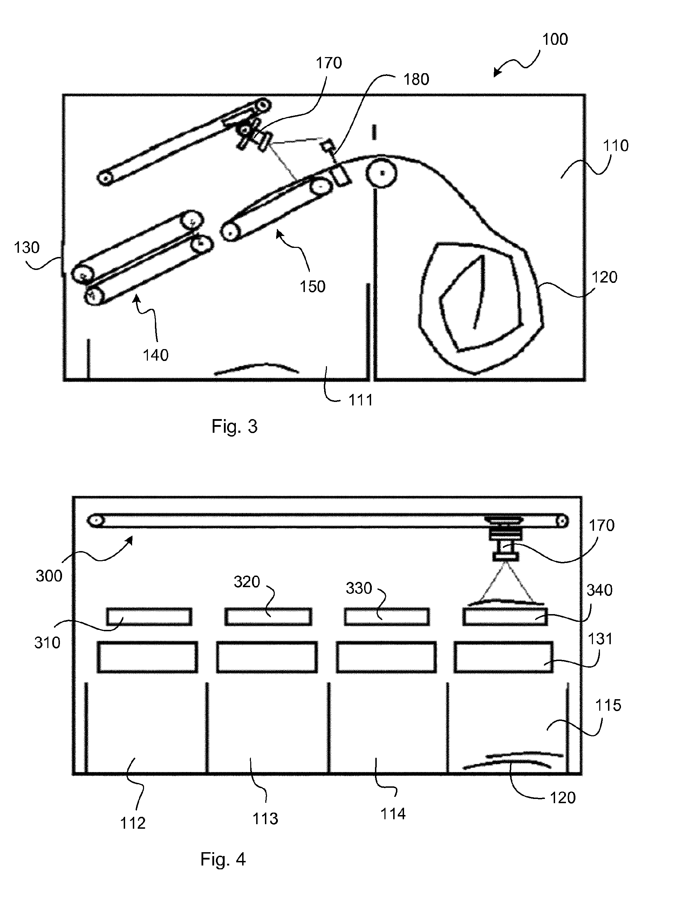

Figure 3 illustrates a sectional view of an embodiment of the present invention in a third position. Indeed, in Figure 3, the camera 170 is in its third position, i.e. configured to monitor the position of the cutter 180. The camera 170 is again tilted to monitor the position of the cutter in a more precice manner.

Figure 4 illustrates another embodiment of the present invention. In this embodiment, the camera 170 is arranged to move along a second belt conveyor 300, which structure is similar to that of the conveyor 200 shown in Figures 1 to 3. The camera 170 is thus able to monitor several parallel belt conveyors 310, 320, 330, 340 used for transporting medication packages as well as the outlets 131 arranged in connection with each parallel belt conveyor. The camera is also then able to monitor several second containers 112, 113, 114, 115 for untaken medication packages 120.

Only advantageous exemplary embodiments of the invention are described in the Figures. It is clear to a person skilled in the art that the invention is not restricted only to the examples presented above, but the invention may vary within the limits of the claims presented hereafter. Some possible embodiments of the invention are described in the dependent claims, and they are not to be considered to restrict the scope of protection of the invention as such.

## Claims

1. A medication dispenser, comprising:
- a first container for holding medication packages that contain medications to be taken at predetermined taking times, and
- conveying means for conveying medication packages from the first container to an outlet of the medication dispenser at the predetermined taking times;
**characterised in that** the medication dispenser comprises:
- a camera for monitoring the operation of the medication dispenser, the camera being arranged to image a plurality of objects at different locations inside the medication dispenser.

2. The medication dispenser according to claim 1, **characterised in that** the medication dispenser comprises moving means for moving the camera between a plurality of positions inside the medication dispenser.

3. The medication dispenser according to claim 2, **characterised in that** the moving means comprises a linear actuator to which the camera is attached.

4. The medication dispenser according to any of the preceding claims, **characterised in that** the medication dispenser comprises rotating means for rotating the camera to different directions.

5. The medication dispenser according to any of the preceding claims, **characterised in that** the optical path between the camera and an object to be imaged comprises an adjustable optical element.

6. The medication dispenser according to any of the claims 2-5, **characterised in that** at a first position the camera is configured to monitor medication packages conveyed on the conveying means.

7. The medication dispenser according to any of the claims 2-6, **characterised in that** at a second position the camera is configured to monitor the content of a second container of the medication dispenser, which second container is arranged to receive untaken medication packages.

8. The medication dispenser according to any of the claims 2-7, **characterised in that** at a third position the camera is configured to monitor the position of a cutter of the medication dispenser.

9. An automated medication administration system comprising a medication dispenser according to any of the claims 1-8 and a medication administration server.

10. A method for monitoring the operation of a medication dispenser that comprises a first container for holding medication packages that contain medications to be taken at predetermined taking times, and conveying means for conveying medication packages from the first container to an outlet of the medication dispenser at the predetermined taking times, **characterised in that** the method comprises:
- monitoring the operation of the medication dispenser by imaging with a camera a plurality of objects at different locations inside the medication dispenser.

11. The method according to claim 10, **characterised in that** the method comprises moving the camera between a plurality of positions inside the medication dispenser, and/or rotating the camera to different directions.

12. The method according to claim 11, **characterised in that** the method comprises monitoring, at a first position, medication packages conveyed on the conveying means.

13. The method according to claim 11 or 12, **characterised in that** the method comprises monitoring, at a second position, the content of a second container of the medication dispenser, which second container is arranged to receive untaken medication packages.

14. The method according to any of the claims 11-13, **characterised in that** the method comprises monitoring, at a third position, the position of a cutter of the medication dispenser.

15. The method according to any of the claims 10-14, **characterised in that** the method comprises, in a situation where the dispenser cannot solve a problem:
- taking an image of the unsolved problem,
- sending the image to a server,
- receiving the solution from the server, and
- self-checking to approve the solution.
